# EUROPÄISCHE PATENTSCHRIFT

(11) **EP 0 709 065 B1**
(45) Veröffentlichungstag und Bekanntmachung des Hinweises auf die Patenterteilung: **26.01.2000**
(21) Anmeldenummer: 95116998.6
(22) Anmeldetag: 27.10.1995
(51) Int. Cl.: A61B 18/12

(54) **Elektrochirurgiegerät und Verfahren zu dessen Betrieb**
Electrosurgical unit and process for its use
Electrobistouri et méthode d'utilisation

(30) Priorität: 31.10.1994 DE 4438978
(43) Veröffentlichungstag der Anmeldung: 01.05.1996
(73) Patentinhaber: Wurzer, Helmut, 80538 München (DE); Mäckel, Rainer, 53639 Königswinter (DE); Liess, Hans Dieter, D-82541 Seeheim/Münsing (DE)
(72) Erfinder: Wurzer, Helmut, D-80538 München (DE); Mäckel, Rainer, D-53639 Königswinter (DE)
(74) Vertreter: Manitz, Finsterwald & Partner

(56) Entgegenhaltungen:
- WO-A-93/03679
- DE-A- 2 504 280
- DE-A- 3 530 335
- DE-A- 3 622 337

## Beschreibung

Die Erfindung betrifft ein Verfahren zum Betrieb eines Elektrochirurgiegerätes nach dem Oberbegriff des Anspruches 1 und ein Elektrochirurgiegerät nach dem Oberbegriff des Patentanspruches 15.

Die Erfindung ist sowohl bei monopolaren als auch bei bipolaren Instrumenten anwendbar.

Mit Hochfrequenzstrom arbeitende Elektrochirurgiegeräte zum Schneiden und/oder Koagulieren menschlichen Gewebes mit Hochfrequenzstrom sind in zahlreichen Ausführungen bekannt. Die vorliegende Erfindung befaßt sich mit solchen Elektrochirurgiegeräten, die entweder nur zur Ausführung eines Hochfrequenzstrom-Schneidvorganges ausgelegt sind oder wahlweise auf die Betriebsart "Schneiden" oder "Koagulieren" umgeschaltet werden können.

Bei der Betriebsart "Schneiden" wird zwischen der Schneidelektrode und dem an geeigneter Stelle mit der Neutralelektrode elektrisch leitend verbundenen Gewebe ein Lichtbogen erzeugt, welcher aus einer in der Frequenz der verwendeten Hochfrequenzspannung entsprechenden Anzahl von Funkenüberschlägen besteht. Bei hoher Leistungsabgabe des Hochfrequenzgenerators findet bei jeder positiven und negativen Halbwelle des Hochfrequenzstromes ein Funkenüberschlag statt. Die Frequenz von Hochfrequenzchirurgiegeräten liegt allgemein in der Größenordnung von 500 kHz. Frequenzen unterhalb von 100 kHz sollten nicht verwendet werden. Ein vernünftiger Frequenzbereich erstreckt sich von ungefähr 300 kHz bis 2 MHz.

Es ist bereits bekannt geworden (DE-OS 25 04 280), die Stärke des Hochfrequenzstromes durch einen automatischen und hinreichend schnellen Regelvorgang so einzustellen, daß jederzeit gerade eine solche Leistung dem Gewebe zugeführt wird, daß einerseits eine für den Schneidvorgang geeignete Erwärmung des Gewebes sichergestellt und andererseits aber das Auftreten von Lichtbögen schädlichen Ausmaßen verhindert wird. Geregelt wird im allgemeinen die Stromstärke, während die vom Hochfrequenzgenerator abgegebene Spannung zumindest innerhalb der beim Betrieb auftretenden Leistungsbereiche im wesentlichen konstantgehalten wird.

Es ist schon versucht worden, die beim Hochfrequenzschneiden auftretende Gleichspannungskomponente oder die durch Verzerrung des Stromverlaufs auftretenden Harmonischen für die Regelung der Leistung des Hochfrequenzgenerators heranzuziehen. Abgesehen von dem relativ hohen technischen Aufwand für die Messung der Harmonischen hat sich bei dieser Meßmethode der Lichtbogen im allgemeinen schon zu stark entwickelt, bevor durch die Messung der Harmonischen eine Gegensteuerung erfolgen kann. Auch die Bestimmung der Zeitdauer der Strompause zwischen zwei Halbwellen kann hier keine Abhilfe schaffen, da diese Strompausen erst bei hohen Leistungen auftreten und der Lichtbogen dann auch schon so stark entwickelt ist, daß das Gewebe geschädigt wird.

Zu ausgeprägte Lichtbögen und damit zu starke, dem Gewebe zugeführte Leistungen führen zu einer Verkohlung des geschnittenen Gewebes, was wiederum den Heilungsprozeß erschwert und verlängert.

Die Aufgabe der Erfindung besteht darin, ein Elektrochirurgiegerät und ein Verfahren zu dessen Betrieb zu schaffen, bei denen einerseits ein problemloser und insbesondere verklebungsfreier Schneidvorgang mittels der Schneidelektrode erzielt wird, gleichwohl aber jedwede über das eigentliche Schneiderfordernis hinausgehende Überhitzung und damit Verkohlung des Gewebes vermieden wird. Insbesondere sollen das erfindungsgemäße Elektrochirurgiegerät und Verfahren eine automatische und schnelle Anpassung der vom Hochfrequenzgenerator abgegebenen Leistung bewirken, wenn beim Schneidvorgang verschiedene Gewebearten (z.B. Muskelfleisch oder Fett) erfaßt werden, die unterschiedliche Leistungsanforderungen haben.

Zur Lösung dieser Aufgabe sind die Merkmale der kennzeichnenden Teile der Ansprüche 1 und 15 vorgesehen.

Die Erfindung beruht auf der Erkenntnis, daß im Falle geringer Leistungen bei einer ersten Berührung zwischen der Schneidelektrode und dem menschlichen oder tierischen Gewebe zunächst nur ein Ohm'scher oder kapazitiver Kontakt zwischen Elektrode und Gewebe vorliegt, wo noch keine Abweichungen von der Sinusform auftreten, und daß bei zunehmender Leistung zunächst nur Überschläge bei einer Sorte von Halbwellen jeder Periode, vorzugsweise der positiven Halbwellen auftreten. Dies resultiert aus den unterschiedlichen Bedingungen, wie Feldverlauf, Temperatur, Austrittsarbeit an der Schneidelektrode und am Gewebe. Im Zeitverlauf des Hochfrequenzstroms sind die Überschläge als kurzzeitige Erhöhungen bzw. Spitzen zu erkennen. Gleichzeitig kann sich die Spannung an der Funkenstrecke entsprechend verringern, wenn keine Konstantspannungscharakteristik verwendet wird. In der jeweils anderen, vorzugsweise der negativen Halbwelle einer Periode treten zunächst bei relativ geringer Leistungszuführung noch keine wesentlichen Abweichungen von der vorgegebenen Sinusform auf. Erst mit weiter zunehmender Leistungszufuhr kommen auch bei den anderen, vorzugsweise negativen Halbwellen zunächst einzelne und dann immer mehr Funkenüberschläge vor.

Das bevorstehende Auftreten eines schädliche Ausmaße annehmenden Lichtbogens kann also auch schon bei relativ geringer Ausgangsleistung des Hochfrequenzgenerators dadurch festgestellt werden, daß einzelne Überschläge innerhalb der positiven Halbwelle ermittelt werden. Die Anzahl dieser auch als Mikrolichtbögen zu bezeichnenden Funkenüberschläge läßt sich mit einer hohen Dynamik sehr schnell bestimmen. Die Bestimmung der Stärke des Lichtbogens kann somit durch eine Zählung der während einer vorbestimmten Anzahl von Perioden auftretenden Funkenüberschlägen oder Mikrolichtbögen erfolgen. Erfindungsgemäß wird also die Intensität des Lichtbogens bzw. der Funkenüberschläge automatisch auf einen einen einwandfreien Schneidvorgang ohne Überhitzungsgefahr gewährleistenden Wert reduziert.

Vorteilhafte Weiterbildungen des erfindungsgemäßen Verfahrens sind durch die Ansprüche 2 bis 14 gekennzeichnet, während besonders bevorzugte Ausführungsformen des erfindungsgemäßen Elektrochirurgiegerätes durch die Ansprüche 16 bis 30 definiert sind.

Die Erfindung wird im folgenden beispielsweise anhand der Zeichnung beschrieben; in dieser zeigt:
- Fig. 1: ein Übersichtsschaltbild eines Hochfrequenzchirurgiegerätes mit einem hinsichtlich seiner Ausgangsleistung geregelten Hochfrequenzgenerator,
- Fig. 2: ein Blockschaltbild einer Ausführungsform einer erfindungsgemäßen Leistungsmeßvorrichtung und Regelstufe bei einem Hochfrequenzchirurgiegerät nach Fig. 1 und
- Fig. 3: eine weitere Ausführungsform einer erfindungsgemäßen Leistungsmeßvorrichtung mit der gleichen Regelstufe, wie sie in Fig. 2 dargestellt ist.

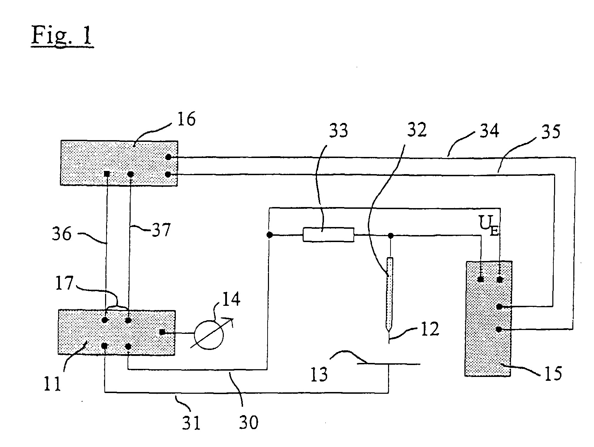

Nach Fig. 1 speist ein beispielsweise mit einer Frequenz von 500 kHz arbeitender Hochfrequenzgenerator 11 über Leitungen 30, 31, in denen zur Gleichspannungsentkopplung Kondensatoren vorgesehen sein könnten, ein hochfrequenzchirurgisches Schneidinstrument 32 mit einer Schneidelektrode 12 bzw. eine Neutralelektrode 13, die an geeigneter Stelle des Körpers eines zu behandelnden Patienten elektrisch leitend anzubringen ist.

Am Hochfrequenzgenerator 11 ist ein Einstellknopf 14 für die Auswahl einer Leistungsgrundeinstellung vorgesehen. Mittels dieses Einstellknopfes 14 kann der Chirurg eine bestimmte Stärke des durchzuführenden Schneidvorganges vorbestimmen, wobei die erfindungsgemäße Regelung so ausgebildet ist, daß sie als Maximalwert diese voreingestellte Leistung berücksichtigt, sie jedoch nach den Erfordernissen des behandelten Gewebes in der erfindungsgemäßen Weise mehr oder weniger reduziert.

In die Zuleitung 30 zur Schneidelektrode 12 ist ein Meßwiderstand 33 eingeschaltet, von dessen Enden eine Spannung U_{E} abgegriffen ist, die für den zur Schneidelektrode 12 fließenden Hochfrequenzstrom repräsentativ und an den Eingang einer erfindungsgemäßen Leistungsmeßvorrichtung 15 angelegt ist.

Der Ausgang der Leistungsmeßvorrichtung 15 ist über Leitungen 34, 35 an den Eingang einer Regelstufe 16 angeschlossen, die aus der festgestellten Ist-Leistung ein Regelsignal erzeugt, welches über Leitungen 36, 37 einen Leistungsregeleingang 17 des Hochfrequenzgenerators 11 so beaufschlagt, daß der Hochfrequenzgenerator 11 auf die Ausgangsleitungen 30, 31 die für das gerade behandelte Gewebe optimale Leistung abgibt. Der Hochfrequenzgenerator 11 soll vorzugsweise mit im wesentlichen konstanter Spannung arbeiten, während zur Leistungsanpassung der Strom geregelt wird.

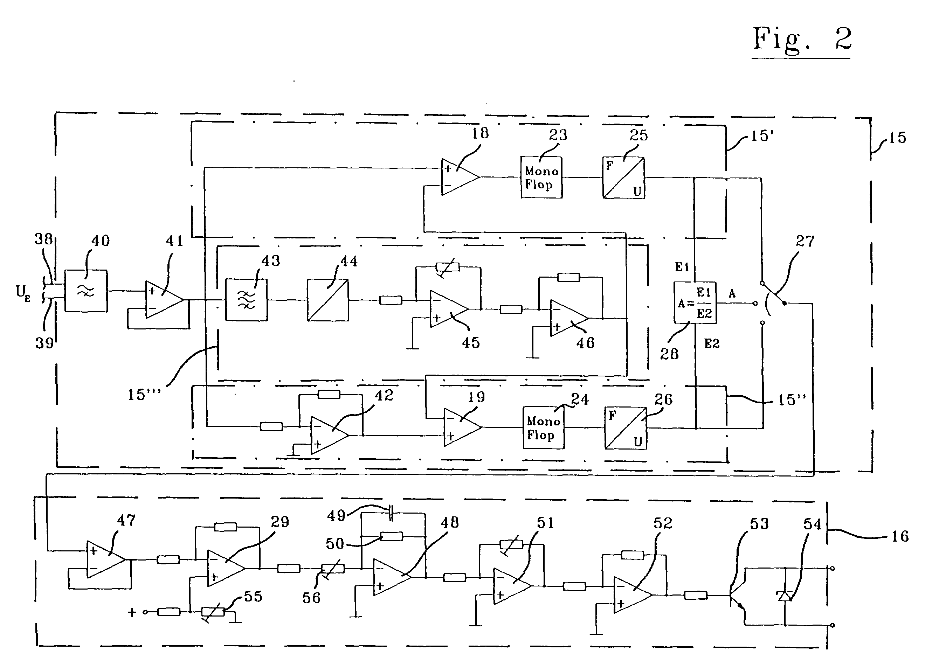

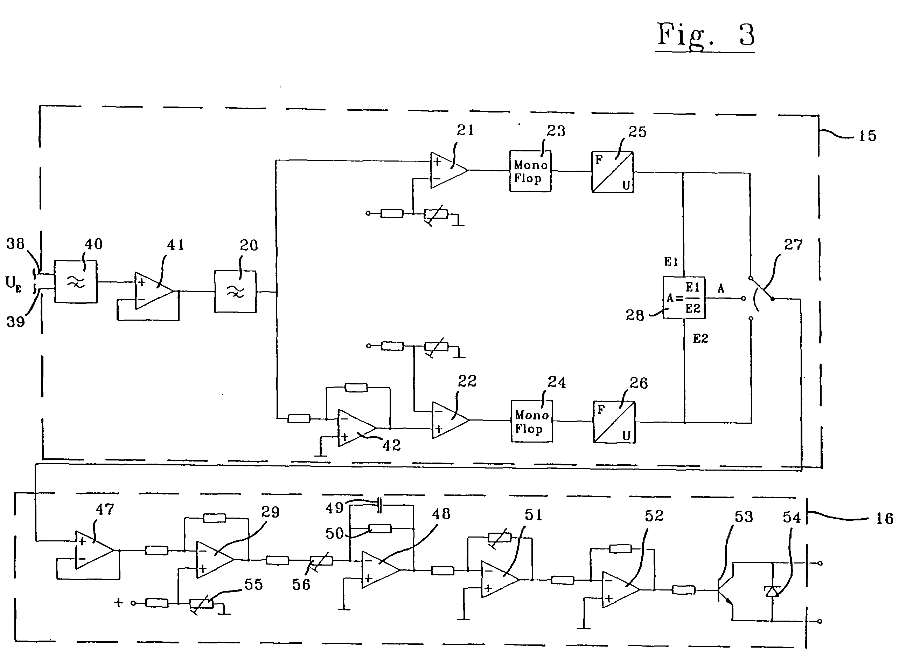

Die erfindungsgemäße Ausbildung der Leistungsmeßvorrichtung 15 und der Regelstufe 16 wird im folgenden anhand der Fig. 2 und 3 im einzelnen beschrieben.

Die dem Strom durch den Meßwiderstand 33 nach Fig. 1 proportionale Spannung U_{E} liegt nach Fig. 2 an Eingangsklemmen 38, 39 der Leistungsmeßvorrichtung 15 an. Sie beaufschlagen einen Hochpaß 40, der bei einer Frequenz des Hochfrequenzgenerators 11 von 500 kHz beispielsweise auf eine Grenzfrequenz von 10 kHz abgestimmt ist. An den Ausgang des Hochpasses 40 ist ein Spannungsfolger 41 angeschlossen, dessen Aufgabe darin besteht, Rückwirkungen der Leistungsmeßvorrichtung 15 auf den Patientenstromkreis zu verhindern.

An den Ausgang des Spannungsfolgers 41 sind parallel zueinander zwei Funkenzählstufen 15' bzw. 15" und eine Referenzwerterzeugungsstufe 15"' angelegt.

Die Funkenzählstufe 15' weist einen Komparator 18 auf, dessen Plus-Eingang an den Ausgang des Spannungsfolgers 41 angeschlossen ist und welcher ein Monoflop 23 beaufschlagt, an das wiederum ein Frequenz-Spannungswandler 25 angeschlossen ist.

Die Funkenzählstufe 15" enthält eine vom Ausgang des Spannungsfolgers 41 beaufschlagte Invertierungsstufe 42, welche das Vorzeichen des Eingangssignals umkehrt. Der Ausgang der Invertierungsstufe 42 liegt am Plus-Eingang eines weiteren Komparators 19, dem ebenfalls ein Monoflop 24 folgt, an das ein Frequenz-Spannungswandler 26 angeschlossen ist.

Die Referenzwert-Erzeugungsstufe 15"' enthält einen an den Spannungsfolger 41 angeschlossenen Bandpaß 43, der auf die Frequenz des Hochfrequenzgenerators von 500 kHz abgestimmt ist und dem eine Gleichrichtungs-Effektivwertbildungsstufe 44 folgt, an die wiederum ein Verstärker 45 angeschlossen ist. Der Ausgang des Verstärkers 45 ist an eine Invertierungsstufe 46 angelegt, an deren Ausgang ein Referenzsignal anliegt, welches dem über mehrere Perioden der Hochfrequenzspannung gemittelten Effektivwert der Hochfrequenzspannung multipliziert mit einem vorbestimmten Faktor entspricht.

Dieses Referenzsignal ist an die Minus-Eingänge der Komparatoren 18 bzw. 19 angelegt.

Aufgrund der beschriebenen Schaltung wertet die Referenzwerterzeugungsstufe 15' die positiven, die Referenzwerterzeugungsstufe 15" die negativen Halbwellen der Hochfrequenzspannung aus.

Wenn eine positive Halbwelle des Hochfrequenzstroms am Plus-Eingang des Komparators 18 ein größeres Signal als den am Minus-Eingang anliegenden Referenzwert erzeugt, gibt der Komparator 18 an das Monoflop 23 einen Impuls ab, welcher dieses veranlaßt, einen Rechteckimpuls (TTL-Signal) zu erzeugen, dessen zeitliche Länge nicht größer ist als die zeitliche Länge einer Halbwelle der Hochfrequenzspannung.

Entsprechend löst eine negative Halbwelle über die Invertierungsstufe 42 am Komparator 19 ein Ausgangssignal aus, wenn die Amplitude der Halbwelle so groß ist, daß das Signal am Plus-Eingang des Komparators 19 den am Minus-Eingang anliegenden Referenzwert übersteigt. Entsprechend wird das Monoflop 24 gesetzt, so daß es einen Rechteckimpuls (TTL-Signal) mit einer Länge kleiner als der Länge der Halbwellen der Hochfrequenzspannung abgibt.

Je nach dem, wie viele positive oder negative Halbwellen das Monoflop 23 bzw. 24 setzen, entsteht am Ausgang der Monoflops 23, 24 ein Signal von höherer oder niedrigerer Frequenz, welches in den Frequenz-Spannungswandlern 25, 26 in ein Spannungssignal umgewandelt wird. Die betreffende Ausgangsspannung liegt einerseits jeweils einem Kontakt eines Dreistellungs-Wahlschalters 27 und andererseits an jeweils einem Eingang einer Verknüpfungsstufe 28 an, deren Ausgang mit dem dritten Kontakt des Dreistellungs-Wahlschalters 27 verbunden ist. Der Mittelkontakt des Dreistellungs-Wahlschalters 27 liegt seinerseits am Eingang der Regelstufe 16 an.

In der Regelstufe 16 ist zunächst ein Spannungsfolger 47 vorgesehen, der wieder eine Rückkopplung auf die vorangehende Leistungsmeßvorrichtung 15 verhindern soll. Ihm folgt eine Differenzstufe 29, deren Pluseingang als Sollwert eine für die konstant zu haltende Anzahl von Funkenüberschlägen repräsentative Regelspannung zuugeführt ist, die durch einen Regelwiderstand 55 veränderbar ist. An die Differenzstufe 29 ist über einen Regelwiderstand 56 ein PI-Regler 48 angeschlossen, dessen Rückkopplungskondensator 49 und Rückkopplungswiderstand 50 so gewählt sind, daß zusammen mit dem Regler 56 sich eine Zeitkonstante im Millisekunden-Bereich ergibt. Bevorzugt liegen die Regelzeiten des PI-Reglers 48 zwischen 0,5 und 10 ms.

Die Ausgangsspannung des PI-Reglers 48 wird in einem Verstärker 51 auf eine gewünschte Signalstärke verstärkt und über eine Invertierungsstufe 52 an einen Bipolartransistor 53 angelegt, der als Senke für den Regeleingang 17 des Hochfrequenzgenerators 11 dient und über die Leitungen 36, 37 nach Fig. 1 an den Regeleingang 17 des Hochfrequenzgenerators 11 angelegt wird.

Die in den Ausgangskreis des Bipolartransistors 53 gelegte Zenerdiode 54 dient dazu, den Transistor vor Überspannung zu schützen.

Die Referenzwerterzeugungsstufe 15"' nach Fig. 2 definiert somit einen dynamischen Schwellenwert, der immer um einen festen Faktor größer ist als die mittlere Amplitude des Meßsignals. Dieser Schwellen- oder Referenzwert dient dazu zu entscheiden, ob ein zu zählender Funkenüberschlag stattgefunden hat oder nicht. Ist die momentane Stromamplitude größer als der Schwellenwert und somit größer als die mit einem festen Faktor multiplizierte mittlere Stromamplitude, so liegt ein zu berücksichtigender Funkenüberschlag vor. Um dies zu überprüfen, werden sowohl der Schwellwert wie auch die Meßgröße den Komparatoren 18 bzw. 19 zugeführt. Ist der Meßwert größer als der Schwellenwert, so liefert der Komparator 18 bzw. 19 eine positive Ausgangsspannung. Ist der Meßwert kleiner als der Schwellenwert, so liegt am Ausgang eine negative Spannung (bzw. Massepotential) an, und es erfolgt keine Setzung der anschließenden Monoflops 23 bzw. 24.

Da die zeitliche Ausdehnung der Überschläge im Verhältnis zur Periodendauer der Hochfrequenzschwingung kurz und undefiniert ist, entstehen am Ausgang kurze positive Spannungsimpulse. Um diesen Impulsen eine definierte Form zu geben, werden sie dem Monoflop 23 bzw. 24 zugeführt, deren Ausgangsimpulsbreite (Länge des Ausgangsimpulses) geringer als die halbe Periodendauer der Grundschwingung ist. Die so aufbereiteten Komparatorsignale werden nun den Frequenz-Spannungswandlern 25 bzw. 26 zugeführt, welche eine Umsetzung der Impulsanzahl pro Zeiteinheit in eine analoge, der Zahl der Überschläge proportionale Spannung vornehmen. Diese Spannung dient als Regelgröße zum Konstanthalten der Stärke des Lichtbogens zwischen der Schneidelektrode 12 und der Neutralelektrode 13. Wichtig ist hierbei, daß diese Spannung monoton mit der eingekoppelten Leistung ist. Erfindungsgemäß werden also weder die Amplitude noch der zeitliche Verlauf der Stromverzerrungen ausgewertet, sondern nur die Häufigkeit der Funkenüberschläge.

Diese Spannung wird im Sollwertkomparator 29 mit einem Sollwert verglichen, dem PI-Regler 48 zugeführt und schließlich in den Hochfrequenzgenerator 11 zurückgekoppelt. Ist die Zahl der Funkenüberschläge und somit der Strom bzw. die Spannung zu groß, so wird die Ausgangsleistung des Generators über die Rückkopplung reduziert. Ist die Anzahl der Funkenüberschläge zu gering, so wird die Ausgangsleistung des Hochfrequenzgenerators 11 dementsprechend erhöht.

Die Wirkungsweise der Schaltung nach Fig. 1 und 2 ist wie folgt:

Nach Einschalten des Hochfrequenzgenerators 11 wird das Schneidinstrument 32 mit der Schneidelektrode 12 dem Gewebe des Patienten genähert, der an anderer Stelle elektrisch leitend mit der Neutralelektrode 13 verbunden ist. Hierbei fließt zunächst ein Ohm'scher Strom mit Sinusform, welcher über die Leistungsmeßvorrichtung 15 und die Regelstufe 16 noch keine Leistungsbegrenzung des Hochfrequenzgenerators 11 auslöst.

Wenn mit zunehmender Leistung während einiger positiver Halbwellen Funkenüberschläge stattfinden, entstehen während dieser Halbwellen Stromspitzen, die, wenn sie dazu führen, daß das Signal am Plus-Eingang des Komparators 18 größer wird als am Referenzeingang (-), das Monoflop 23 setzen und am Ausgang des Frequenz-Spannungswandlers 25 eine entsprechende Spannung hervorrufen.

Bei je mehr Halbwellen Funkenüberschläge und damit Stromspitzen entstehen, um so höher wird die Ausgangsspannung des Frequenz-Spannungswandlers 25.

Bei noch höheren Leistungen bilden sich auch bei den negativen Halbwellen Funken, wodurch über den Komparator 19 das Monoflop 24 entsprechend gesetzt und am Ausgang des Frequenz-Spannungswandlers 26 eine der Zahl der festgestellten Stromspitzen bzw. Funken entsprechende Spannung anliegt.

Nach Fig. 2 ist der Wahlschalter 27 an den Ausgang des Frequenzspannungswandlers 25 angelegt, so daß dessen Ausgangssignal, welches der in einem vorbestimmten Zeitraum festgestellten Funkenzahl proportional ist, am Eingang der Regelstufe 16 anliegt, die daraus ein Regelsignal U_{A} bildet, welches die Leistung des Hochfrequenzgenerators 11 zurückregelt, wenn die während einer vorbestimmten Zahl von Perioden des Hochfrequenzstroms auftretende Zahl von Funkenüberschlägen geringfügig überschritten wird. Umgekehrt wird die Leistung des Hochfrequenzgenerators 11 hinaufgeregelt, wenn zu wenig Funken festgestellt werden.

Durch Umlegen des Wahlschalters 27 in die in Fig. 2 untere Position wird das Ausgangssignal des Frequenz-Spannungswandlers 26 an die Regelstufe 16 angelegt, so daß nunmehr die Zahl der innerhalb der vorbestimmten Zahl von Perioden bei den negativen Halbwellen auftretenden Funken für das an den Eingang der Regelstufe 16 angelegte Eingangssignal verantwortlich ist. Je nach dem, wie viele Funken bzw. Stromspitzen bei den negativen Halbwellen von der Funkenzählstufe 15" gezählt werden, regelt das Ausgangssignal U_{A} der Regelstufe 16 die Leistung des Hochfrequenzgenerators 11 zurück oder hinauf. Bei einem Konstant-Spannungshochfrequenzgenerator 11 entspricht dies einer Zurückregelung des Effektivwertes des Hochfrequenzstroms.

Befindet sich der Wahlschalter 27 nach Fig. 2 in seiner Mittelstellung, so ist für die Beaufschlagung der Regestufe 16 das Ausgangssignal A der Verknüpfungsschaltung 28 maßgebend, welches beispielsweise den Quotienten E1/E2 der beiden Ausgangssignale der Frequenz-Spannungswandler 25 bzw. 26 bildet.

Beim Ausführungsbeispiel nach Fig. 3 bezeichnen gleiche Bezugszahlen entsprechende Bauelemente wie in Fig. 2. Die Schaltung ist gegenüber Fig. 2 dadurch wesentlich vereinfacht, daß die Referenzwerterzeugungsstufe 15"' entfällt.

Stattdessen ist an den Ausgang des Spannungsfolgers 41 ein Hochpaß 20 angeschlossen, welcher auf eine etwas höhere Frequenz als die des Hochfrequenzgenerators abgestimmt ist. Beträgt die Frequenz des Hochfrequenzgenerators 500 kHz soll der Hochpaß 20 beispielsweise auf eine Frequenz von 600 kHz abgestimmt sein.

Auf diese Weise passieren den Hochpaß 20 lediglich die wesentliche steileren Stromspitzen, die bei Vorliegen eines Funkens während einer Halbwelle auftreten. Die bei Funkenbildung vom Hochpaß 20 abgegebenen Signale sind parallel an einen Komparator 21 und - über die Invertierungsstufe 42 an einen Komparator 22 angelegt, die an ihrem Referenzeingang jeweils von einer vorzugsweise einstellbaren Referenzspannung beaufschlagt sind. Die Spannung ist frei wählbar und bestimmt den Schwellenwert, bei dessen Überschreiten durch die Amplitude Funkenüberschläge gezählt werden.

Auf diese Weise entstehen an den Ausgängen der Komparatoren 21, 22 bei übermäßiger Funkenbildung während einer positiven oder negativen Halbwelle entsprechende Signale wie beim Ausführungsbeispiel nach Fig. 2, welche dann in der gleichen Weise über die Monoflops 23, 24, die Frequenz-Spannungswandler 25, 26 verarbeitet und über den Wahlschalter 27 bzw. die Verknüpfungsschaltung 28 und den Wahlschalter 27 an die Regelstufe 16 angelegt sind.

Die Bildung der Referenzspannung für die Komparatoren 18, 19 nach Fig. 2 erfolgt also dynamisch durch Auswertung des Effektivwertes des zur Schneidelektrode 12 fließenden Stromes, wobei im Verstärker 45 eine Multiplikation mit einem festen Faktor erfolgt, wodurch erst bei einer vorbestimmten Amplitude der Stromspitzen die Schwellen der Komparatoren 18 bzw. 19 überschritten werden.

Die Spannung am Ausgang der Frequenz-Spannungswandler 25, 26 ist monoton zur Zahl der Funkenüberschläge. Demgegenüber wird der Referenzwert für die Komparatoren 21, 22 nach Fig. 3 durch eine Gleichspannung vorgegeben, während der Plus-Eingang durch den Hochpaß 20 mit einer Grenzfrequenz beaufschlagt wird, die höher als die Frequenz des Hochfrequenzgenerators 11 ist.

Anstelle des Frequenz-Spannungswandlers 25 bzw. 26 kann auch ein digitaler Zähler verwendet werden, was die Realisierung der Regelung mit einem digitalen Regelkreis ermöglicht.

Die Mittenfrequenz des Bandpaßfilters 43 nach Fig. 2 entspricht der Betriebsfrequenz des Hochfrequenzgenerators. Durch eine ausreichend hohe Güte des Bandpaßfilters 43 wird sichergestellt, daß nur die Grundschwingung und keine Harmonischen übertragen werden.

Beim Ausführungsbeispiel nach Fig. 3 wird anstelle der Bestimmung der mittleren Stromamplitude die Grundschwingung mit Hilfe des Hochpasses 20 aus dem Eingangssignal herausgefiltert. Die Harmonischen bleiben hier alle erhalten. Dies erlaubt die Festlegung eines statischen Schwellenwertes für die Entscheidung, ob ein Überschlag vorliegt oder nicht. Dieser Schwellenwert kann einfach mit Hilfe eines Spannungsteilers aus der Betriebsspannung erzeugt werden.

## Patentansprüche

1. Verfahren zum Betrieb eines Elektrochirurgiegerätes mit einem einen Leistungsregeleingang (17) aufweisenden Hochfrequenzgenerator (11), der eine vorzugsweise veränderbare (14) Leistungsgrundeinstellung aufweist und an den eine Schneidelektrode (12) sowie eine Neutralelektrode (13) angeschlossen bzw. anschließbar sind, die mit einer solchen Hochfrequenzwechselspannung und einem solchen Hochfrequenzwechselstrom beaufschlagbar sind, daß zumindest während eines Teils der Perioden des HochfrequenzWechselstroms wenigstens ein Funkenüberschlag erfolgt, wobei an die Elektroden (12, 13) eine Leistungsbestimmungsvorrichtung (15) angeschlossen ist, die eine Regelstufe (16) beaufschlagt, welche an den Leistungsregeleingang (17) des Hochfrequenzgenerators (11) angelegt ist,
dadurch gekennzeichnet, daß die Anzahl der Funkenüberschläge innerhalb einer vorbestimmten Mehrzahl von Perioden bestimmt und durch Veränderung der Ausgangsleistung des Hochfrequenzgenerators (11) auf einen konstanten Wert eingeregelt wird, der unterhalb des Doppelten der vorbestimmten Mehrzahl liegt.

2. Verfahren nach Anspruch 1,
dadurch gekennzeichnet, daß der konstante Wert unterhalb der vorbestimmten Mehrzahl liegt.

3. Verfahren nach Anspruch 1 oder 2,
dadurch gekennzeichnet, daß die vorbestimmte Mehrzahl von Perioden zwischen 100 und 10 000 liegt.

4. Verfahren nach Anspruch 3,
dadurch gekennzeichnet, daß die vorbestimmte Mehrzahl von Perioden zwischen 1000 und 10 000 liegt.

5. Verfahren nach Anspruch 4,
dadurch gekennzeichnet, daß die vorbestimmte Mehrzahl von Perioden zwischen 3000 und 8000 liegt.

6. Verfahren nach Anspruch 5,
dadurch gekennzeichnet, daß die vorbestimmte Mehrzahl von Perioden bei etwa 5000 liegt.

7. Verfahren nach einem der vorangehenden Ansprüche,
dadurch gekennzeichnet, daß der eingeregelte konstante Wert 1 bis 20 % der vorbestimmten Mehrzahl beträgt.

8. Verfahren nach Anspruch 7,
dadurch gekennzeichnet, daß der eingeregelte konstante Wert 1 bis 10 % der vorbestimmten Mehrzahl beträgt.

9. Verfahren nach Anspruch 8,
dadurch gekennzeichnet, daß der eingeregelte Wert 1 bis 2 % der vorbestimmten Mehrzahl beträgt.

10. Verfahren nach einem der vorhergehenden Ansprüche,
dadurch gekennzeichnet, daß als Maß für das Auftreten eines Funkens die Überschreitung bzw. Unterschreitung eines vorbestimmten Schwellenstromwertes verwendet wird, wobei die Hochfrequenzspannung zumindest im wesentlichen konstantgehalten werden soll.

11. Verfahren nach einem der vorhergehenden Ansprüche,
dadurch gekennzeichnet, daß für die Regelung nur die während der positiven Halbwellen oder die während der negativen Halbwellen oder während aller Halbwellen innerhalb der vorbestimmten Mehrzahl von Perioden festgestellten Funkenüberschläge herangezogen werden.

12. Verfahren nach einem der vorhergehenden Ansprüche,
dadurch gekennzeichnet, daß bei Heranziehung der während aller Halbwellen innerhalb der vorbestimmten Mehrzahl von Perioden festgestellten Funkenüberschläge die Zahl der während der positiven und der negativen Halbwellen festgestellten Zahlen von Funkenüberschlägen mathematisch miteinander verknüpft werden.

13. Verfahren nach Anspruch 12,
dadurch gekennzeichnet, daß die mathematische Verknüpfung dadurch geschieht, daß die festgestellten Anzahlen von Funkenüberschlägen während der positiven und negativen Halbwellen addiert, subtrahiert, multipliziert oder dividiert werden, wobei die Division nur bei Verwendung eines monopolaren Instrumentes in Betracht kommt.

14. Verfahren nach einem der vorhergehenden Ansprüche,
dadurch gekennzeichnet, daß der konstante Wert der Anzahl von Funkenüberschlägen einstellbar ist.

15. Elektrochirurgiegerät mit einem einen Leistungsregeleingang (17) aufweisenden Hochfrequenzgenerator (11), der eine vorzugsweise veränderbare (14) Leistungsgrundeinstellung aufweist und an den eine Schneidelektrode (12) sowie eine Neutralelektrode (13) angeschlossen bzw. anschließbar sind, die mit einer solchen Hochfrequenzwechselspannung und einem solchen Hochfrequenzwechselstrom beaufschlagbar sind, daß zumindest während eines Teils der Perioden des Hochfrequenzwechselstroms wenigstens ein Funkenüberschlag erfolgt, wobei an die Elektroden (12, 13) eine Leistungsbestimmungsvorrichtung (15) angeschlossen ist, die eine Regelstufe (16) beaufschlagt, welche an den Leistungsregeleingang (17) des Hochfrequenzgenerators (11) angelegt ist,
dadurch gekennzeichnet, daß die Leistungsmeßvorrichtung wenigstens eine Funkenzählstufe (15', 15") aufweist, die die Funkenzahl innerhalb einer vorbestimmten Mehrzahl von Perioden während der positiven oder während der negativen Halbwellen oder während aller Halbwellen bestimmt und die Regelstufe (16) mit einem für die festgestellte Funkenzahl oder eine mathematische Verknüpfung der festgestellten Funkenzahlen repräsentativen Signals beaufschlagt, und daß die Regelstufe (16) daraus ein Regelsignal erzeugt, welches die Ausgangsleistung des Hochfrequenzgenerators (11) im Sinne einer Konstanthalung der innerhalb der vorbestimmten Mehrzahl von Perioden vorhandenen Funkenzahl auf einen Wert, der unterhalb des Doppelten der vorbestimmten Mehrzahl liegt, regelt.

16. Elektrochirurgiegerät nach Anspruch 15,
dadurch gekennzeichnet, der Wert unterhalb der vorbestimmten Mehrzahl liegt.

17. Elektrochirurgiegerät nach Anspruch 15 oder 16,
dadurch gekennzeichnet, daß als Maß für das Vorliegen eines Funkens die Überschreitung bzw. Unterschreitung eines vorbestimmten Schwellenstromwertes während der Halbwelle einer Periode verwendet wird.

18. Elektrochirurgiegerät nach Anspruch 17,
dadurch gekennzeichnet, daß der Hochfrequenzgenerator (11) eine Konstantspannungscharakteristik aufweist.

19. Elektrochirurgiegerät nach Anspruch 18,
dadurch gekennzeichnet, daß aus den den Stromschwellenwert über- bzw. unterschreitenden Meßwerten ein entsprechendes Frequenzsignal gebildet und in eine die Regelstufe (16) beaufschlagendes Spannungssignal umgewandelt wird.

20. Elektrochirurgiegerät nach Anspruch 19,
dadurch gekennzeichnet, daß in der Funkenzählvorrichtung (15) wenigstens ein Komparator (18, 19) vorgesehen ist, dem das aktuelle Strommeßsignal und der über eine vorbestimmte Zahl von Perioden ermittelte Stromeffektivwert zugeführt sind, wobei der Komparator (18, 19) einen Ausgangsimpuls abgibt, wenn das aktuelle Strommeßsignal die vom Stromeffektivwert abgeleitete Komparatorschwelle über- bzw. unterschreitet, oder in der Funkenzählstufe (15) ein Hochpaß (20) vorgesehen ist, der lediglich die steilere Anstiege aufweisenden Stromspitzen durchläßt, welche bei Funkenüberschlägen bestimmter Intensität auftreten.

21. Elektrochirurgiegerät nach Anspruch 20,
dadurch gekennzeichnet, daß an den Hochpaß (20) wenigstens ein Komparator (21, 22) angeschlossen ist, der eine Schwelle für die Stromspitzen darstellt.

22. Elektrochirurgiegerät nach Anspruch 21,
dadurch gekennzeichnet, daß die Schwelle für die Stromspitzen einstellbar ist.

23. Elektrochirurgiegerät nach einem der Ansprüche 20 bis 22,
dadurch gekennzeichnet, daß an den Komparator (18, 19; 21, 22) jeweils ein Monoflop (23, 24) mit einer deutlich kürzeren Schaltzeit als die halbe Periode des Hochfrequenzstromes angelegt ist, dem ein Frequenz-Spannungswandler (25, 26) folgt.

24. Elektrochirurgiegerät nach einem der Ansprüche 15 bis 23,
dadurch gekennzeichnet, daß für die positiven und negativen Halbwellen des Hochfrequenzstromes eine separate Funkenzählung erfolgt.

25. Elektrochirurgiegerät nach Anspruch 24,
dadurch gekennzeichnet, daß entweder nur die während der positiven oder negativen Halbwellen auftretenden Funken gezählt werden oder eine mathematische Verknüpfung beider Funkenarten erfolgt.

26. Elektrochirurgiegerät nach Anspruch 24 oder 25,
dadurch gekennzeichnet, daß am Ausgang des Frequenz-Spannungswandlers (25, 26) ein Wahlschalter (27) und/oder eine mathematische Verknüpfungsschaltung (28) vorgesehen sind.

27. Elektrochirurgiegerät nach Anspruch 26,
dadurch gekennzeichnet, daß der Wahlschalter drei Stellungen aufweist.

28. Elektrochirurgiegerät nach einem der Ansprüche 24 bis 27,
dadurch gekennzeichnet, daß die mathematische Verknüpfung in der Quotientenbildung des separat bei den positiven Halbwellen und bei den negativen Halbwellen erzeugten Zählsignals besteht.

29. Elektrochirurgiegerät nach einem der Ansprüche 15 bis 28,
dadurch gekennzeichnet, daß die Regelstufe (16) eine Differenzstufe (29) umfaßt, an der der konstante Wert von Funkenüberschlägen eingestellt werden kann.

30. Elektrochirurgiegerät nach Anspruch 29,
dadurch gekennzeichnet, daß die Differenzstufe (29) einstellbar ist.

## Claims

1. Method of operating an electrosurgical device having a radio-frequency generator (11) with a power control input (17) and a preferably variable (14) basic power setting, wherein a cutting electrode (12) and also a neutral electrode (13) are connected or can be connected to the radio frequency generator and can be subjected to such a radio-frequency alternating voltage and to such a radio-frequency alternating current that at least one sparkover occurs during a part of the period of the radio-frequency alternating current, and wherein a power determining device (15) is connected to the electrodes (12, 13) and acts on a regulating stage (16) which is connected to the power regulating input (17) of the radio-frequency generator (11), characterized in that the number of sparkovers within a predetermined plural number of periods is determined and is regulated, by changing the output power of the radio-frequency generator (11), to a constant value which lies below twice the predetermined number.

2. Method in accordance with claim 1, characterized in that the constant value lies below the predetermined number.

3. Method in accordance with claim 1 or claim 2, characterized in that the predetermined number of periods lies between 100 and 10,000.

4. Method in accordance with claim 3, characterized in that the predetermined number of periods lies between 1000 and 10,000.

5. Method in accordance with claim 4, characterized in that the predetermined number of periods lies between 3000 and 8000.

6. Method in accordance with claim 5, characterized in that the predetermined number of periods lies at approximately 5000.

7. Method in accordance with one of the preceding claims, characterized in that the regulated constant value amounts to 1 to 20 % of the predetermined number.

8. Method in accordance with claim 7, characterized in that the regulated constant value amounts to 1 to 10 % of the predetermined number.

9. Method in accordance with claim 8, characterized in that the regulated constant value amounts to 1 to 2 % of the predetermined number.

10. Method in accordance with one of the preceding claims, characterized in that as a measure for the occurrence of a sparkover use is made of a predetermined threshold current value being exceeded and/or fallen short off, with the radio-frequency voltage being kept at least substantially constant.

11. Method in accordance with one of the preceding claims, characterized in that for the regulation use is made only of the sparkovers which are detected during the positive half-waves, or during the negative half-waves, or during all half-waves within the predetermined number of periods.

12. Method in accordance with one of the preceding claims, characterized in that when using the number of sparkovers determined during all half-waves within the predetermined number of periods, the count of the numbers of sparkovers detected during the positive and the negative half-waves are mathematically linked together.

13. Method in accordance with claim 12, characterized in that the mathematical linking takes place in such a way that the numbers of sparkovers determined during the positive and the negative half-waves are added, subtracted, multiplied or divided, with division only being considered when using a monopolar instrument.

14. Method in accordance with one of the preceding claims, characterized in that the constant value of the number of sparkovers is adjustable.

15. Electrosurgical apparatus having a radio-frequency generator (11) with a power regulating input (17) and a preferably variable (14) basic power setting, wherein a cutting electrode (12) and also a neutral electrode (13) are connected to or can be connected to the radio-frequency generator and can be subjected to such a radio-frequency alternating voltage and to such a radio-frequency alternating current that at least one sparkover occurs during a part of the periods of the radio-frequency alternating current and wherein a power determining device (15) is connected to the electrodes (12, 13) and acts on a regulating stage (16) which is connected to the power regulating input (17) of the radio-frequency generator (11), characterized in that the power measuring device has at least one sparkover counting stage (15', 15") which determines the number of sparkovers during a predetermined plural number of periods during the positive half-waves, or during the negative half-waves, or during all half-waves, and applies to the regulating stage (16) a signal representative of the determined number of sparkovers, or a mathematical combination of the number of sparkovers detected; and in that the regulating stage (16) generates from this a regulating signal which regulates the output power of the radio-frequency generator (11) in the sense of keeping constant the number of sparkovers present within the predetermined plural number of periods at a value which lies beneath twice the predetermined plural number.

16. Electrosurgical apparatus in accordance with claim 15, characterized in that the value lies below the predetermined plural number.

17. Electrosurgical apparatus in accordance with claim 15 or claim 16, characterized in that the exceeding of, or the falling short of, a predetermined threshold current value during the half-wave of a period is used as a measure for the presence of an arc.

18. Electrosurgical apparatus in accordance with claim 17, characterized in that the radio-frequency generator (11) has a constant voltage characteristic.

19. Electrosurgical apparatus in accordance with claim 18, characterized in that a corresponding frequency signal is formed from the measured values which exceed the current threshold value and/or fall short off the current threshold value and is converted into a voltage signal which acts on the regulating stage (16).

20. Electrosurgical apparatus in accordance with claim 19, characterized in that at least one comparator (18, 19) is provided in the sparkover counting device (15) to which the actual current measurement signal and the effective current value determined over a predetermined number of periods are supplied, with the comparator (18, 19) transmitting an output pulse when the actual current measurement signal exceeds or falls short off the comparator threshold derived from the effective current value, or a high-pass filter (20) is provided in the sparkover counting stage (15) which only permits current peaks having steeper rise times to pass, which occur with sparkovers of specific intensity.

21. Electrosurgical apparatus in accordance with claim 20, characterized in that at least one comparator (21, 22) is connected to the high-pass filter (20) and represents a threshold for the current peaks.

22. Electrosurgical apparatus in accordance with claim 21, characterized in that the threshold for the current peaks is adjustable.

23. Electrosurgical apparatus in accordance with one of the claims 20 to 22, characterized in that in each case a monoflop (23, 24) with a substantially shorter switching time than the half period of the radio-frequency current is connected to the comparator (18, 19; 21, 22), with the monoflop being followed by a frequency/voltage converter (25, 26).

24. Electrosurgical apparatus in accordance with one of the claims 15 to 23, characterized in that a separate sparkover count takes place for the positive and negative half-waves of the radio-frequency current.

25. Electrosurgical apparatus in accordance with claim 24, characterized in that either only the sparkovers occuring during the positive or negative half-waves are counted or a mathematical combination of both sparkover types takes place.

26. Electrosurgical apparatus in accordance with claim 24 or claim 25, characterized in that a selection switch (27) and/or a mathematical logic circuit (28) are provided at the output of the frequency/voltage converter (25, 26).

27. Electrosurgical apparatus in accordance with claim 26, characterized in that the selection switch has three positions.

28. Electrosurgical apparatus in accordance with one of the claims 24 to 27, characterized in that the mathematical combination consists of quotient formation of the count signals separately generated for the positive half-waves and for the negative half-waves.

29. Electrosurgical apparatus in accordance with one of the claims 15 to 28, characterized in that the regulating stage (16) has a difference forming stage (29) at which the constant value for the sparkovers can be set.

30. Electrosurgical apparatus in accordance with claim 29, characterized in that the difference stage (29) is adjustable.

## Revendications

1. Procédé pour le fonctionnement d'un bistouri électrique comportant un oscillateur haute fréquence (11) qui comprend une entrée de régulation de puissance (17) et qui présente un réglage de puissance de base (14) de préférence variable auquel sont connectées ou susceptibles d'être connectées une électrode de coupe (12) ainsi qu'une électrode neutre (13), qui peuvent être alimentées sous une tension alternative haute fréquence et avec un courant alternatif haute fréquence, tels que du moins pendant une partie des périodes du courant alternatif haute fréquence au moins un claquage a lieu, un dispositif déterminant la puissance (15) étant connecté aux électrodes (12, 13), lequel attaque un étage de régulation (16) qui est appliqué à l'entrée de régulation de puissance (17) de l'oscillateur haute fréquence (11), caractérisé en ce que le nombre des claquages à l'intérieur d'une pluralité de périodes prédéterminées est déterminé et régulé à une valeur constante par modification de la puissance de sortie de l'oscillateur haute fréquence (11), ladite valeur étant inférieure au double de la pluralité prédéterminée.

2. Procédé selon la revendication 1, caractérisé en ce que la valeur constante est inférieure à la pluralité prédéterminée.

3. Procédé selon l'une ou l'autre des revendications 1 et 2, caractérisé en ce que la pluralité prédéterminée de périodes est comprise entre 100 et 10000.

4. Procédé selon la revendication 3, caractérisé en ce que la pluralité prédéterminée de périodes est comprise entre 1000 et 10000.

5. Procédé selon la revendication 4, caractérisé en ce que la pluralité prédéterminée de périodes est comprise entre 3000 et 8000.

6. Procédé selon la revendication 5, caractérisé en ce que la pluralité prédéterminée de périodes est d'environ 5000.

7. Procédé selon l'une quelconque des revendications précédentes, caractérisé en ce que la valeur régulée constante est comprise entre 1 et 20 % de la pluralité prédéterminée.

8. Procédé selon la revendication 7, caractérisé en ce que la valeur régulée constante est comprise entre 1 et 10 % de la pluralité prédéterminée.

9. Procédé selon la revendication 8, caractérisé en ce que la valeur régulée constante est comprise entre 1 et 2 % de la pluralité prédéterminée.

10. Procédé selon l'une quelconque des revendications précédentes, caractérisé en ce que l'on utilise, en tant que valeur pour l'apparition d'un claquage, le passage au-dessus ou au-dessous d'une valeur de courant seuil prédéterminée, la tension haute fréquence devant être maintenue du moins sensiblement constante.

11. Procédé selon l'une quelconque des revendications précédentes, caractérisé en ce que pour la régulation on exploite uniquement les claquages constatés pendant les demi-ondes positives ou pendant les demi-ondes négatives ou pendant toutes les demi-ondes à l'intérieur de la pluralité prédéterminée de périodes.

12. Procédé selon l'une quelconque des revendications précédentes, caractérisé en ce qu'en exploitant les claquages constatés pendant toutes les demi-ondes à l'intérieur de la pluralité de périodes, les nombres des claquages constatés pendant les demi-ondes positives et négatives sont enchaînés par voie mathématique.

13. Procédé selon la revendication 12, caractérisé en ce que l'enchaînement mathématique s'effectue par le fait que les nombres de claquages constatés pendant les demi-ondes positives et négatives sont additionnés, soustraits, multipliés ou divisés, la division s'appliquant uniquement lorsque l'on utilise un instrument monopolaire.

14. Procédé selon l'une quelconque des revendications précédentes, caractérisé en ce que la valeur constante du nombre des claquages est réglable.

15. Bistouri électrique comportant un oscillateur haute fréquence (11) qui comprend une entrée de régulation de puissance (17) et qui présente un réglage de puissance de base (14) de préférence variable auquel sont connectées ou susceptibles d'être connectées une électrode de coupe (12) ainsi qu'une électrode neutre (13), qui peuvent être alimentées en une tension alternative haute fréquence et avec un courant alternatif haute fréquence, tels que du moins pendant une partie des périodes du courant alternatif haute fréquence au moins un claquage a lieu, un dispositif déterminant la puissance (15) étant connecté aux électrodes (12, 13), lequel attaque un étage de régulation (16) qui est appliqué à l'entrée de régulation de puissance (17) de l'oscillateur haute fréquence (11), caractérisé en ce que le dispositif de mesure de puissance présente au moins un étage de comptage des claquages (15', 15") qui détermine le nombre de claquages à l'intérieur d'une pluralité prédéterminée de périodes pendant les demi-ondes positives ou pendant les demi-ondes négatives ou pendant toutes les demi-ondes et qui alimente l'étage de régulation (16) avec un signal représentatif du nombre de claquages constaté ou d'un enchaînement mathématique des nombres de claquages constatés, et en ce que l'étage de régulation (16) produit à partir de celui-ci un signal de régulation qui règle la puissance de sortie de l'oscillateur haute fréquence (11) dans le sens visant à maintenir le nombre de claquages présents à l'intérieur de la pluralité prédéterminée de périodes à une valeur constante qui est inférieure au double de la pluralité prédéterminée.

16. Bistouri électrique selon la revendication 15, caractérisé en ce que la valeur est inférieure à la pluralité prédéterminée.

17. Bistouri électrique selon l'une ou l'autre des revendications 15 et 16, caractérisé en ce que le passage au-dessous ou au-dessus d'une valeur de courant seuil prédéterminée pendant la demi-onde d'une période est utilisé en tant que valeur pour la présence d'un claquage.

18. Bistouri électrique selon la revendication 17, caractérisé en ce que l'oscillateur haute fréquence (11) présente une caractéristique de tension constante.

19. Bistouri électrique selon la revendication 18, caractérisé en ce qu'à partir des valeurs de mesure supérieures ou inférieures à la valeur de courant seuil est formé un signal de fréquence correspondant et celui-ci est converti en un signal de tension qui attaque l'étage de régulation (16).

20. Bistouri électrique selon la revendication 19, caractérisé en ce qu'au moins un comparateur (18, 19) est prévu dans le dispositif de comptage de claquages (15), auquel sont amenés le signal de mesure de courant actuel et la valeur de courant effective détectée sur un nombre prédéterminé de périodes, le comparateur (18, 19) émettant une impulsion de sortie lorsque le signal de mesure de courant actuel est inférieur ou supérieur à un seuil de comparateur dérivé de la valeur de courant effective, ou qu'un filtre passe-haut (20) est prévu dans l'étage de comptage de claquages (15), ledit filtre laissant passer uniquement les pointes de courant qui présentent les montées plus raides et qui apparaissent en cas de claquages d'une certaine intensité.

21. Bistouri électrique selon la revendication 20, caractérisé en ce qu'au moins un comparateur (21, 22) est branché au filtre passe-haut (20), ledit comparateur représentant un seuil pour les pointes de courant.

22. Bistouri électrique selon la revendication 21, caractérisé en ce que le seuil pour les pointes de courant est réglable.

23. Bistouri électrique selon l'une quelconque des revendications 20 à 22, caractérisé en ce qu'au comparateur (18, 19 ; 21, 22) est appliquée une bascule monostable respective (23, 24) dont le temps de communication est nettement plus court que la moitié de la période du courant haute fréquence, ladite bascule étant suivie par un transformateur fréquence/tension (25, 26).

24. Bistouri électrique selon l'une quelconque des revendications 15 à 23, caractérisé en ce que pour les demi-ondes positives et négatives du courant haute fréquence a lieu un comptage de claquages séparé.

25. Bistouri électrique selon la revendication 24, caractérisé en ce que soit les claquages apparaissant pendant les demi-ondes positives ou négatives sont comptés, soit encore un enchaînement mathématique des deux types de claquages a lieu.

26. Bistouri électrique selon l'une ou l'autre des revendications 24 et 25, caractérisé en ce qu'à la sortie du transformateur fréquence/tension (25, 26) est prévu un commutateur de sélection (27) et/ou un circuit d'enchaînement mathématique (28).

27. Bistouri électrique selon la revendication 26, caractérisé en ce que le commutateur de sélection présente trois positions.

28. Bistouri électrique selon l'une quelconque des revendications 24 à 27, caractérisé en ce que l'enchaînement mathématique consiste à former le quotient des signaux de comptage générés séparément pour les demi-ondes positives et pour les demi-ondes négatives.

29. Bistouri électrique selon l'une quelconque des revendications 15 à 28, caractérisé en ce que l'étage de régulation (16) comprend un étage différenciateur (29) au niveau duquel la valeur constante de claquages peut être réglée.

30. Bistouri électrique selon la revendication 29, caractérisé en ce que l'étage différenciateur (29) est réglable.
